# EUROPEAN PATENT SPECIFICATION

(11) **EP 1 596 960 B1**
(45) Date of publication and mention of the grant of the patent: **16.12.2009**
(21) Application number: 04713478.8
(22) Date of filing: 21.02.2004
(51) Int. Cl.: B01D 39/16, B03C 3/28

(54) **AIR CLEANNESS FILTER**
LUFTREINIGUNGSFILTER
FILTRE DE PURIFICATION D'AIR

(30) Priority: 21.02.2003 KR 2003010899
(43) Date of publication of application: 23.11.2005
(73) Proprietor: Kim, Euiwoong, Seoul 156-848 (KR)
(72) Inventor: Kim, Euiwoong, Seoul 156-848 (KR)
(74) Representative: Winkler, Andreas Fritz Ernst
(86) International application number: PCT/KR2004/000361
(87) International publication number: WO 2004/073833

(56) References cited:
- EP-A- 0 199 528
- EP-A1- 1 197 252
- WO-A1-99/65593
- WO-A1-02/081055
- KR-A- 99 068 474
- KR-A- 2002 040 550
- PATENT ABSTRACTS OF JAPAN vol. 1996, no. 01, 31 January 1996 (1996-01-31) & JP 07 241491 A (TOYOBO CO LTD), 19 September 1995 (1995-09-19)
- PATENT ABSTRACTS OF JAPAN vol. 017, no. 344 (E-1390), 29 June 1993 (1993-06-29) & JP 05 048283 A (FUJITSU LTD), 26 February 1993 (1993-02-26)
- DATABASE WPI Week 199149 Derwent Publications Ltd., London, GB; AN 1991-356988 XP002377764 & JP 03 238011 A (KURARAY CHEM CO LTD) 23 October 1991 (1991-10-23)

## Description

The present invention relates to an air cleanness filter, and more particularly to an air cleanness filter charged with static electricity.

As industries have been developed, fossil fuels are excessively used so that great amounts of gas are exhausted from industrial facilities. In addition, a great amount of dust or particles are generated due to an expansion of urban-based facilities, thereby creating the environmental problem of air pollution.

Typically, a mechanical filtering method and an electrostatic filtering method have been used for collecting such exhaust gases or particles. The mechanical filtering method uses fiber filters, such as woven fabrics or non-woven fabrics, which do not filter particles having a micro size (for example, particles having a size less than 10 micron). In addition, particles are accumulated in the fiber filters so that a pressure loss is increased and a life span thereof is shortened.

The electrostatic filtering method uses a ratio frequency discharge capable of obtaining high particulate-collecting efficiency. However, such electrostatic filtering method causes high power consumption and raises problems in view of stability, repair and maintenance. In addition, such electrostatic filtering method may generate a great amount of ozone, causing difficulty in breathing and a headache.

Korean Patent No. 310274 discloses a non-power electrostatic filter having superior collecting efficiency. According to the non-power electrostatic filter, a fiber filter, such as woven fabric or non-woven fabric, is formed by using high-polymer resin including polypropylene or polyethylene. In addition, the fiber filter is charged with static electricity by means of a high-voltage generator in order to collect particles by using static electricity.

However, such a non-power electrostatic filter uses woven fabric or non-woven fabric in the same manner as a mechanical filter used in the conventional mechanical filtering method, allowing air to flow vertically to the non-power electrostatic filter, so that a pressure less is increased. In addition, when collecting air by using a fan, power consumption may be increased and loud noise may be generated. If a thickness of the non-power electrostatic filter becomes increased in order to improve filtering efficiency, the pressure loss may be more increased, the non-power electrostatic filter presents a limitation in filtering efficiency. Due to such limitation in filtering efficiency, the non-power electrostatic filter cannot filter micro particles, so the non-power electrostatic filter cannot purify stench generated from acid gas or alkaline gas contained in the micro particles.

JP-A-07241491 discloses an electrostatically charged cylindrical filter made of corrugated polypropylene.

The present invention has been made in view of the above-mentioned problems of the prior art, and it is an object of the present invention to provide an air cleanness filter capable of improving filtering efficiency.

In order to accomplish the above object, the present invention provides an air cleanness filter according to claim 1.

The foregoing and other objects, features and advantages of the present invention will become more apparent from the following detailed description when taken in conjunction with the accompanying drawings in which:
FIG. 1 is a perspective view showing an air cleanness filter according to one embodiment of the present invention; and
FIG. 2 is an enlarged sectional view of an air cleanness filter according to one embodiment of the present invention.

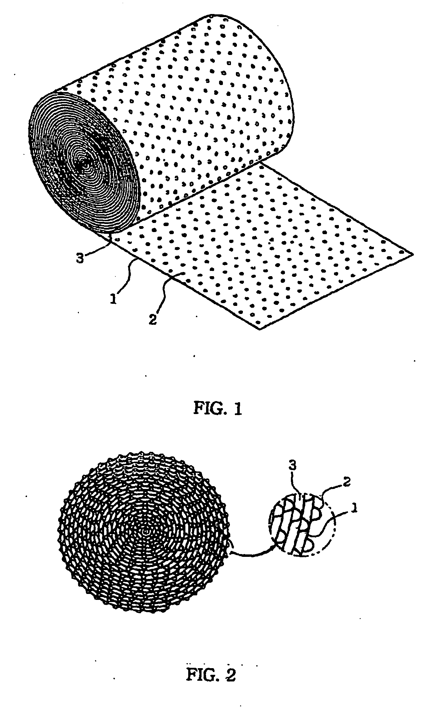

As shown in FIGS. 1 and 2, an air cleanness filter of the present invention includes a high-polymer synthetic resin film 1, such as polypropylene or polyethylene, which is electrically charged by means of a charging device such as a 10 to 40 KV corona discharge device and is formed with a plurality of protrusions 2. The film 1 is wound in the form of a cylinder so as to provide an air passage 3 in parallel to an air flow direction.

A thickness of the film 1 is preferably about 0.1 to 1 mm for maintaining strength thereof. A height of the protrusions 2 can be set within a range of about -0.1 to 5 mm depending on the condition of air, amount of air (intake air), or an R.P.M of a fan. The height of the protrusions 2 may be set according to a width of the film 1 so that a pressure loss may be adjustable.

That is, narrower the width of the film 1 becomes narrow, lower the height of the protrusion 2 becomes low. In addition, if the width of the film 1 becomes enlarged, the height of the protrusion 2 becomes larger.

In addition, since the air cleanness filter is generally accommodated in a case, the height of the protrusions 2 may be determined after setting the width of the film 1 according to configuration of the case including a volume and a shape of the case.

The protrusions 2 are irregularly formed on the film 1 in order to allow air passing through the air passage 3 to irregularly collide with the protrusions 2 while forming bypass air passages causing air to collide with each other, thereby improving air filtering efficiency.

According to the present invention, air is introduced into the air passage 3 in parallel to an air flow direction so that the air cleanness filter can filter fine particles contained in such air while purifying stench of gas contained in fine particles. In order to efficiently purify stench of acid gas or alkaline gas contained in fine particles, a porous deodorant, such as activated carbon or zeolite, and a sterilizer, such as Ag or TiO₂, capable of sterilizing bacteria are preferably dispersed on the film 1 when extruding the film 1. In addition, it is preferred to fabricate a wide film and cut the wide film into several films having a predetermined size in use.

According to the present invention, the air cleanness filter is accommodated in the case and a fan is installed at a rear portion of the case so as to intake air to be cleaned in parallel to the air passage 3. When air passes through the air passage 3, static electricity of the film 1, which is permanently charged in the film 1, may react with particles so that the particles are filtered by means of the air cleanness filter and stench of gas contained in the particles is removed. Since air to be cleaned flows in parallel to the air passage 3, the pressure loss becomes reduced. In addition, since a traveling distance of air in the air passage 3 becomes increased, electrification between the film 1 and the particles may be improved, thereby increasing filtering efficiency.

According to the present invention, if the width of the film 1 becomes narrow, the height of the protrusion 2 becomes low, and, if the width of the film 1 becomes enlarged, the height of the protrusion 2 becomes high, so that an air cleaner can be designed in various sizes.

While this invention has been described in connection with what is presently considered to be the most practical and preferred embodiment, it is to be understood that the invention is not limited to the disclosed embodiment and the drawings, but, on the contrary, it is intended to cover various modifications and variations within the scope of the appended claims.

As can be seen from the foregoing, according to the present invention, a high-polymer synthetic resin film is electro-statically charged and is formed with a plurality of protrusions. In addition, the high-polymer synthetic resin film is wound in the form of a cylinder so as to provide an air passage in parallel to an air flow direction in such a manner that air may flow through the air passage in parallel to the air passage. Moreover, the protrusions are irregularly formed on the film. Accordingly, the air cleanness filter of the present invention can improve filtering efficiency. In addition, the air cleanness filter of the present invention can filter micro particles, so that stench from acid gas or alkaline gas contained in such particles can be effectively purified.

Furthermore, since the height of the protrusions can be adjusted according to the width of the filter, it is possible to design an air cleaner with various sizes while reducing the pressure loss, power consumption, and noise.

## Claims

1. An air cleanness filter comprising:
a film made of high-polymer synthetic resin and charged with static electricity; and
a plurality of protrusions formed on the film, wherein the film is wound in a cylinder shape so as to provide an air passage in parallel to an air flow direction,
wherein the protrusions are irregularly formed on the film.

2. The air cleanness filter as claimed in claim 1, wherein heights of the protrusions are adjustable according to a width of the film.

3. The air cleanness filter as claimed in claim 1, wherein a porous deodorant and a sterilizer are sprayed on the film.

## Patentansprüche

1. Luftreinigungsfilter, welcher umfasst:
einen Film, der aus hoch-polymerem synthetischen Harz hergestellt ist und mit statischer Elektrizität beladen ist; und
eine Vielzahl an auf dem Film gebildeten Vorsprüngen, wobei der Film in eine Zylinderform gewickelt ist, um einen Luftdurchfluss parallel zu einer Luftfließrichtung bereitzustellen,
wobei die Vorsprünge unregelmäßig auf dem Film gebildet sind.

2. Luftreinigungsfilter nach Anspruch 1, wobei Höhen der Vorsprünge entsprechend einer Breite des Films einstellbar sind.

3. Luftreinigungsfilter nach Anspruch 1, wobei ein poröses Desodorisierungsmittel und ein Sterilisationsmittel auf den Film gesprüht sind.

## Revendications

1. Filtre de nettoyage d'air comprenant:
un film réalisé en une résine synthétique à hauts polymères et chargé d'électricité statique; et
plusieurs protubérances formées sur le film, où le film est enroulé sous forme de cylindre de sorte à procurer un passage d'air parallèlement à une direction d'écoulement de l'air,
où les protubérances sont formées de manière irrégulière sur le film.

2. Filtre de nettoyage d'air tel que revendiqué dans la revendication 1, dans lequel les hauteurs des protubérances sont réglables selon la largeur du film.

3. Filtre de nettoyage d'air tel que revendiqué dans la revendication 1, dans lequel un déodorant poreux et un stérilisateur sont pulvérisés sur le film.
